(19) Europäisches Patentamt
European Patent Office
Office européen des brevets

(11) **EP 2 158 939 B1**

(12) **EUROPÄISCHE PATENTSCHRIFT**

(45) Veröffentlichungstag und Bekanntmachung des Hinweises auf die Patenterteilung:
**04.06.2014   Patentblatt 2014/23**

(51) Int Cl.:
***A61N 5/10*** *(2006.01)*

(21) Anmeldenummer: **09162690.3**

(22) Anmeldetag: **15.06.2009**

(54) **Verfahren und Vorrichtung zur Auswahl eines Bestrahlungsplans sowie Bestrahlungsanlage**

Method and device for selecting an irradiation plan and irradiation assembly

Procédé et dispositif de sélection d'un plan de rayonnement et installation de rayonnement

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(30) Priorität: **29.08.2008   DE 102008044901**

(43) Veröffentlichungstag der Anmeldung:
**03.03.2010   Patentblatt 2010/09**

(73) Patentinhaber: **Siemens Aktiengesellschaft 80333 München (DE)**

(72) Erfinder:
• **Rietzel, Eike, Dr.**
**64289, Darmstadt (DE)**
• **Vidal, Marie**
**91054, Erlangen (DE)**

(56) Entgegenhaltungen:
**US-A1- 2005 201 516     US-A1- 2007 076 846**
**US-A1- 2008 109 013**

EP 2 158 939 B1

**Beschreibung**

Die Erfindung betrifft ein Verfahren und eine Vorrichtung zur Auswahl eines Bestrahlungsplans sowie eine Bestrahlungsanlage mit einer derartigen Vorrichtung. Eine derartige Vorrichtung bzw. ein derartiges Verfahren findet üblicherweise Einsatz im Rahmen einer Strahlentherapie, insbesondere im Rahmen einer Partikeltherapie.

Im Vorfeld einer Strahlentherapie ist es notwendig, einen Bestrahlungsplan zu bestimmen. Diese Bestimmung ist mitunter schwierig, da sich die interne Anatomie eines Patienten mit der Zeit verändern kann. Beispielsweise können Zielvolumina innerhalb des Abdomens ihre Lage von Tag zu Tag ändern oder im Verlauf mehrerer Tage oder Wochen. Ein typisches Organ, das oftmals einer Positionsänderung unterliegt, ist die Prostata. So können z.B. die neben der Prostata gelegene Blase und das neben der Prostata gelegene Rektum einen Einfluss auf die Lage und die Form der Prostata haben, je nach Füllungszustand der Blase und/oder des Rektums.

Eine Möglichkeit, diese Veränderungen zu berücksichtigen, ist die Verwendung von Sicherheitssäumen. Bei der Bestrahlungsplanung werden derartige Sicherheitssäume so gewählt, dass eine interne Verschiebung/Verformung des Zielvolumens berücksichtigt wird. Obwohl mit diesen Sicherheitssäumen die negativen Auswirkungen einer Lageänderung des Zielvolumens gemildert werden können, können diese Sicherheitssäume zu einer Bestrahlung von benachbarten, kritischen Strukturen führen, wie beispielsweise der Blase oder des Rektums.

In der Dissertation von Nikoghosyan A., "Evaluation of the therapeutical potential of heavy ion therapy for patients with locally advanced prostate cancer", Oktober 2004, Medizinische Fakultät Heidelberg, ist das Konzept offenbart, aus einem Tumorvolumen (GTV - engl.: "gross tumor volume") ein klinisches Zielvolumen (CTV - engl.: "clinical target volume) durch Erweitern um Säume zu bestimmen. Ein Planungszielvolumen (PTV - engl.: "planning target volume") wird hieraus mit zusätzlichem Sicherheitssaum bestimmt, um unter anderem eine Organbewegung zu berücksichtigen. Die Größe des Zielvolumens wurde an weiteren, nachfolgenden CT-Datensätzen erneut ausgewertet.

Aus der Schrift US 2005/0201516 A1 ist das Konzept bekannt, für ein Zielvolumen mehreren Bestrahlungspläne mit unterschiedlichen Sicherheitssäumen zu berechnen (MMODS für engl.: "multiple-margin optimization with daily selection"). Ein Anwender kann dann in Echtzeit aus einer Vielzahl von optimierten Bestrahlungsplänen mit unterschiedlichen Sicherheitssäumen denjenigen auswählen, um eine beobachtete Veränderung in der Größe oder Position des Tumors oder den Tumor umgebenden Strukturen zu berücksichtigen.

Die US2007/0076846 offenbart den Gegenstand der Präambel des Anspruchs 1.

Es ist die Aufgabe der Erfindung, ein Verfahren bzw. eine Vorrichtung zur Auswahl eines Bestrahlungsplans bereitzustellen, die es jeweils erlauben, schnell und einfach einen Bestrahlungsplan auszuwählen, der eine Lageänderung des zu bestrahlenden Zielvolumens in guter Qualität berücksichtigt. Weiterhin ist es die Aufgabe der Erfindung, eine Bestrahlungsanlage mit einer derartigen Vorrichtung anzugeben.

Die Aufgabe der Erfindung wird gelöst durch ein Verfahren gemäß Anspruch 1, durch eine Vorrichtung gemäß Anspruch 7 sowie durch eine Bestrahlungsanlage gemäß Anspruch 11.

Ausgestaltungen und Vorteile, wie sie bei der nachfolgenden Beschreibung im Zusammenhang mit einem der Verfahren beschrieben werden, gelten sinngemäß ebenso für eine der Vorrichtungen und umgekehrt.

Das erfindungsgemäße Verfahren zur Auswahl eines Bestrahlungsplans weist folgende Schritte auf:

In einer ersten Phase

- Erfassung von mehreren Planungsdatensätzen, in welchen ein zu bestrahlendes Zielvolumen mit unterschiedlicher Lage in einem Zielobjekt abgebildet ist, und
- Erstellung eines Bestrahlungsplans für jeden dieser Planungsdatensätze,

In einer der ersten Phase nachfolgenden zweiten Phase

- Aufzeichnen eines Verifikationsdatensatzes,
- Vergleichen des Verifikationsdatensatzes mit den mehreren Planungsdatensätzen hinsichtlich einer Ähnlichkeit,
- Auswählen eines Planungsdatensatzes aus den mehreren Planungsdatensätzen, welcher die größte Ähnlichkeit mit dem Verifikationsdatensatz aufweist,
- Auswählen des Bestrahlungsplans, der dem ausgewählten Planungsdatensatz zugeordnet ist.

Die Planungsdatensätze werden folglich im Vorfeld in einer ersten Phase aufgezeichnet, vorzugsweise zu unterschiedlichen Zeitpunkten, so dass sich das zu bestrahlende Zielvolumen in den Planungsdatensätzen in verschiedenen Positionen und/oder Verformungszuständen befindet. Je mehr Planungsdatensätze aufgezeichnet werden, desto eher wird die Bandbreite der üblicherweise auftretenden unterschiedlichen Positionen bzw. Verformungen des Zielvolumens erfasst. Üblicherweise dürften jedoch bereits 3 bis 5 Planungsdatensätze reichen, um einen ausreichenden Ausschnitt aus der Bandbreite der wichtigsten Positions- bzw. Formänderungen des Zielvolumens zu erfassen. Unter den mehreren Planungsdatensätzen können auch Planungsdatensätze vorkommen, in denen das zu bestrahlende Zielvolumen zufälligerweise eine in etwa gleiche Lage oder Verformung aufweist. Dies ist für das Verfahren nicht schädlich, solange die Vielzahl der Planungsdatensätze insgesamt die Bandbreite der wichtigsten Lage- und Formänderungen des Zielvolumens abdeckt.

Für jeden dieser Planungsdatensätze wird im Folgenden ein Bestrahlungsplan erstellt. Die Bestrahlungspläne sind dabei auf die jeweiligen Besonderheiten, die durch die Planungsdatensätze gegeben sind, optimiert. Planungsdatensätze werden üblicherweise mit einem Computertomographen aufgezeichnet.

Wenn dann beispielsweise an einem Tag, an dem eine Bestrahlung geplant ist, ein Verifikationsdatensatz aufgezeichnet wird, der ebenfalls das Zielvolumen abbildet, kann ein Vergleich zwischen dem Verifikationsdatensatz und den Planungsdatensätzen stattfinden. Der Vergleich dient dazu, eine Ähnlichkeit zwischen dem Verifikationsdatensatz und jedem der Planungsdatensätze zu bestimmen, beispielsweise mithilfe eines Ähnlichkeitsmaßes, das jeweils das Ausmaß einer Übereinstimmung zwischen dem Verifikationsdatensatz und einem der Planungsdatensätze angibt.

Üblicherweise wird der Verifikationsdatensatz ebenso wie die Planungsdatensätze das Zielvolumen und die umgebenden Organe/Strukturen abbilden. Es jedoch auch denkbar, dass in dem Verifikationsdatensatz vornehmlich oder lediglich diejenigen Volumina abgebildet werden, welche einen Einfluss auf die Lage des Zielvolumens haben, ohne das Zielvolumen selbst abzubilden.

Der Verifikationsdatensatz kann ebenfalls mit einem Computertomographen aufgezeichnet werden. Es kann jedoch auch eine andere Bildgebungsmodalität als bei den Planungsdatensätzen verwendet werden, beispielsweise ein Cone-Beam-CT, das mit Hilfe eines C-Bogen-Röntgengerätes aufgezeichnet werden kann.

Es ist sogar denkbar, dass der Verifikationsdatensatz lediglich ein 2D-Datensatz ist, beispielsweise ein 2D-Röntgenbild, welches das Zielvolumen abbildet und welches mit den Planungsdatensätzen verglichen wird. Bei CT-Planungsdatensätzen können für den Vergleich passende digital rekonstruierte Radiographien (auch als DRR bezeichnet) erzeugt werden. Alternativ und/oder zusätzlich können zu den CT-Planungsdatensätzen, welche für die Bestrahlungsplanung verwendet werden, auch 2D-Planungsdatensätze aufgezeichnet werden, die dem 2D-Verifikationsbild entsprechen und die für den Vergleich herangezogen werden.

Der Vergleich der Planungsdatensätze mit dem Verifikationsdatensatz hinsichtlich einer Ähnlichkeit bedeutet, dass jeweils vorliegende Unterschiede zwischen dem Verifikationsdatensatzes und einem Planungsdatensatz ermittelt und beurteilt werden.

Als Methoden, ein Maß zur Bewertung der Ähnlichkeit zu ermitteln, stehen verschiedene bekannte mathematische Methoden zur Verfügung, wie beispielsweise eine Summe der Unterschiede im Quadrat (engl: "sum of squared differences"), eine Kreuzkorrelation (engl: "cross correlation"), gegenseitige Informationen (engl: "mutual information"). Es ist aber auch denkbar, in den Datensätzen geometrische Abstände beispielsweise von markanten Strukturen zu ermitteln, und das Ähnlichkeitsmaß mit Hilfe der geometrischen Abstände zu errechnen.

Ebenso ist es auch denkbar, eine Registrierung durchzuführen, um die Ähnlichkeit zwischen dem Verifikationsdatensatz und den Planungsdatensätzen festzustellen. Die Registrierung gibt nämlich die notwendige Transformation an, um einen Planungsdatensatz in den Verifikationsdatensatz überzuführen. Aus dieser Transformationsvorschrift kann ein Maß ermittelt werden, dass die Ähnlichkeit zwischen dem Verifikationsdatensatz und einem der Planungsdatensätzen angibt. Dieses Verfahren kann jedoch je nach verwendeter Registrierungsart rechenintensiv sein im Vergleich zu anderen Verfahren, ein Maß zur Bewertung der Ähnlichkeit zu finden.

Derjenige Planungsdatensatz, der die größte Übereinstimmung oder die größte Ähnlichkeit mit dem Verifikationsdatensatz aufweist, wird daraufhin ausgewählt. Der diesem Planungsdatensatz zugeordnete Bestrahlungsplan ist dann der ausgewählte Bestrahlungsplan, der gegebenenfalls anschließend verwendet werden kann, um die geplante Bestrahlung tatsächlich durchzuführen.

Mit dem Verfahren ist es folglich möglich, einen Bestrahlungsplan auszuwählen, der der spezifischen Situation, die zu dem Zeitpunkt vorliegt, an dem der Verifikationsdatensatz aufgezeichnet wird, am ehesten gerecht wird. Dieser Bestrahlungsplan kann dann, gegebenenfalls nach Überprüfung weiterer Umstände, für eine nachfolgende Bestrahlungssitzung verwendet werden. Es ist jedoch nicht zwingend notwendig, eine Bestrahlung durchzuführen. Beispielsweise können andere Gründe, z.B. eine plötzlich auftretende Unpässlichkeit des Patienten oder andere unvorhergesehene Ereignisse dazu führen, eine Bestrahlung doch nicht wie geplant mit dem ausgewählten Bestrahlungsdatensatz durchzuführen.

Insgesamt verbessert das Verfahren eine adaptive Bestrahlungsplanung und damit die Möglichkeiten zur adaptiven Strahlentherapie, welche die vorliegende Anatomie an einem bestimmten Tag berücksichtigt. Hierdurch ist es möglich, das Ausmaß eines Sicherheitssaumes zu verringern und trotzdem zu gewährleisten, dass das Zielvolumen mit einer Solldosis belegt wird.

Das Verfahren hat den Vorteil, dass zur Bestimmung des zu verwendenden Bestrahlungsplans keinerlei Neuberechnung, Optimierung oder Anpassung eines bestehenden Bestrahlungsplans notwendig ist, wofür gegebenenfalls eine aufwändige Registrierung von Bilddatensätzen durchgeführt werden müsste. Es wird lediglich ein Vergleich zwischen Datensätzen durchgeführt, der direkt zu einem bereits bestehenden Bestrahlungsplan führt, welcher üblicherweise bereits geprüft und für akzeptabel befunden worden ist. Aufwändige Rechenschritte werden dadurch eingespart, wodurch das Verfahren schnell und einfach durchgeführt werden kann. Zudem werden sicherheitskritische Aspekte auf elegante Weise gelöst, die sonst vorhanden wären, da eine Neuberechnung des Bestrahlungsplans bzw. eine Abänderung eines bestehenden Bestrahlungsplans stets die Gefahr birgt, dass der entstehende Bestrahlungsplan nicht mehr den sicherheitsrelevanten Anforderungen genügt und diese Aspekte erneut sichergestellt werden müssten.

Falls gewünscht, kann vor Durchführung des Vergleichs eine Ausrichtung des Verifizierungsdatensatzes und der Planungsdatensätze zueinander durchgeführt werden, wodurch gewährleistet wird, dass beim Vergleich auch korrespondierende Bereiche miteinander verglichen werden. Auf eine derartige Ausrichtung könnte jedoch auch verzichtet werden, wenn der Patient bei der Aufnahme der Planungsdatensätze und bei der Aufnahme des Verifikationsdatensatzes hinreichend genau in gleicher Position positioniert war.

Erfindungsgemäß wird ein Bereich in einem der Planungsdatensätze und/oder in dem Verifikationsdatensatz ausgewählt, wobei der Verifikationsdatensatz mit den mehreren Planungsdatensätzen lediglich in diesem Bereich hinsichtlich der Ähnlichkeit verglichen wird.

Der Bereich umfasst dabei einen Teil der Abbildung des Planungsdatensatzes und/oder des Verifikationsdatensatzes. Bestimmte Bereiche, die für den Vergleich hinsichtlich der Ähnlichkeit irrelevant oder gar schädlich sind, können so ausgeschlossen werden. Zu diesen Bereichen können beispielsweise knöcherne Strukturen gehören, die sich üblicherweise in ihrer Lage von Planungsdatensatz zu Planungsdatensatz nicht oder nicht stark unterscheiden. Knöcherne Strukturen können zwar dafür verwendet werden, eine Ausrichtung zwischen den Planungsdatensätzen und dem Verifikationsdatensatz zu bestimmen, allerdings sind knöcherne Strukturen für die Beurteilung der Ähnlichkeit anderer interner Anatomie weniger relevant.

Organe oder Organabschnitte, die in ihrer Form und Position sehr variabel sind, die aber keinen räumlichen Bezug zu dem Zielvolumen haben, da sie distant vom Zielvolumen liegen und keinen Berührungspunkt zum Zielvolumen haben, können auch bei einem Vergleich hinsichtlich der Ähnlichkeit ausgeschlossen werden. Für die Lage, Position und Form der Prostata sind beispielsweise die benachbarten Organe Blase und Rektum relevant. Weiter entfernt liegende Abschnitte des Kolons haben jedoch so gut wie keinen Einfluss. Wenn der Vergleich hinsichtlich der Ähnlichkeit lediglich in einem bestimmten Bereich durchgeführt wird, können derartige entfernte Strukturen beim Vergleich ausgeschlossen werden, die das Ergebnis eher verfälschen als verbessern würden. In dieser Ausführungsform umfasst der Bereich also zumindest einen Teilbereich in einem der Planungsdatensätze und/oder in dem Verifikationsdatensatz nicht, welcher Teilbereich keinen Berührungspunkt mit dem Zielvolumen hat.

Erfindungsgemäß umfasst der Bereich das Zielvolumen und zumindest einen an das Zielvolumen angrenzenden Bereich vorzugsweise zusätzlich auch einen Eintrittskanal eines Behandlungsstrahls.

Dadurch, dass der Bereich einen an das Zielvolumen angrenzenden Bereich umfassen kann, wird der Tatsache Rechnung getragen, dass oftmals Strukturen und/oder Organe, die an das Zielvolumen angrenzen, die Position und/oder die Form des Zielvolumens beeinflussen. Außerdem sind Grenzen zwischen Strukturen bei der Bestimmung der Ähnlichkeit oftmals von Vorteil, da diese Grenzen charakteristische Unterschiede in den Planungs- und Verifikationsdatensätzen aufweisen können, wohingegen einzelne Organe sich teilweise homogen darstellen können.

Dadurch, dass der Bereich den Eintrittskanal eines Behandlungsstrahls umfassen kann, wird den Bedürfnissen Rechnung getragen, die vor allem im Rahmen der Partikeltherapie auftreten. Während bei einer Bestrahlung mit Photonen der Eintrittskanal weniger wichtig ist, da die Beschaffenheit des Eintrittskanals einen geringen Einfluss auf die im Zielvolumen deponierte Dosis hat, sind die Verhältnisse im Eintrittskanal bei einer Bestrahlung mit Partikeln wichtiger, da dort auftretende Dichteschwankungen einen empfindlichen Einfluss auf die Reichweite des Partikelstrahls haben. Dadurch, dass bei der Beurteilung der Ähnlichkeit zwischen dem Verifikationsdatensatz und den Planungsdatensätzen der Eintrittskanal berücksichtigt wird, kann die Auswahl des passenden Bestrahlungsplans entscheidend verbessert werden.

Es ist auch denkbar, dass der Bereich lediglich einen Bereich umfasst, der mit dem Zielvolumen keinen unmittelbaren Berührungspunkt hat. Beispielsweise könnte der Verifikationsdatensatz eine zweidimensionale fluoroskopische Aufnahme sein. In einer derartigen Aufnahme kann es vorkommen, dass das Zielvolumen, oftmals Weichteilgewebe, nur in schlechter Qualität abgebildet wird. Andere Strukturen, wie beispielsweise Knochen oder das Zwerchfell, können sich in diesen Aufnahmen schärfer darstellen und damit besser identifizierbar sein. Bei Bestrahlung z.B. eines Lungentumors kann es dann mitunter bereits ausreichend sein, die Lage des Zwerchfells zu berücksichtigen und bei dem Vergleich des Verifikationsdatensatzes mit den Planungsdatensätzen heranzuziehen und nicht das Zielvolumen selbst.

In einer anderen vorteilhaften Ausführungsform kann der Bereich zumindest eine an das Zielvolumen angrenzende Struktur, z.B. ein an das Zielvolumen angrenzendes Organ, lediglich teilweise umfassen.

Auch hier wurde erkannt, dass ein an das Zielvolumen angrenzendes Organ nicht notwendigerweise vollständig hinsichtlich einer Ähnlichkeit beurteilt werden muss. Es reicht mitunter bereits aus, lediglich den an das Zielvolumen angrenzenden Bereich des Organs bzw. der Struktur zu berücksichtigen.

In einer vorteilhaften Ausführungsform umfassen die mehreren Planungsdatensätze und/oder der Verifikationsdatensatz, vorzugsweise alle Datensätze, eine zeitliche Dimension. Dies bedeutet, dass in den mehreren Planungsdatensatz in bzw. in dem Verifikationsdatensatz ein Bewegungsverlauf abgebildet ist.

Die Planungsdatensätze können beispielsweise 4D-CT Datensätze sein, der Verifikationsdatensatz ein 4D-Cone-Beam-CT Datensatz. Derartige Datensätze erlauben es, eine Bewegung zu beurteilen, die während einer geplanten Bestrahlungssitzung auftreten kann. Auf diese Weise kann z.B. die Bewegungscharakteristik, die in dem Verifikationsdatensatz vorliegt, mit den unterschiedlichen Bewegungscharakteristiken, die in den Planungsdatensätzen vorliegen, verglichen werden. Dies kann beispielsweise geschehen, indem ein Parameter, welcher die Bewegung charakterisiert, wie beispielsweise eine Amplitude oder eine Frequenz der Bewegung, ermittelt wird, gegebenenfalls zusammen mit einem weiteren Parameter wie dem Zentrum des Zielvolumens oder dessen mittlere Position.

Für eine derartige Anwendung kann die Ähnlichkeit gemeinsam oder separat zwischen verschiedenen Phasen der Bewegung beurteilt werden. Daraufhin kann derjenige Planungsdatensatz ausgewählt werden, der die ähnlichste Charakteristik hinsichtlich der Bewegung aufweist.

In einer besonderen Ausführungsform kann bei dem Vergleich der mehreren Planungsdatensätze mit dem Verifikationsdatensatz eine Sensitivitätskarte verwendet werden. Die Sensitivitätskarte charakterisiert eine Variabilität zwischen den mehreren Planungsdatensätzen bezüglich unterschiedlicher Regionen. Die Sensitivitätskarte kann dabei durch einen Vergleich der mehreren Planungsdatensätze untereinander und/oder mit dem Verifikationsdatensatz ermittelt werden.

Die Sensitivitätskarte, welche aus den Datensätzen selbst ermittelt werden kann, kennzeichnet folglich, welche Bereiche eine starke Variabilität von Planungsdatensatz zu Planungsdatensatz aufweisen und welche Bereiche eher konstant bleiben. Diese Sensitivitätskarte kann dann vorteilhaft bei dem Vergleich des Verifikationsdatensatzes mit den Planungsdatensätzen eingesetzt werden, da nun bekannt ist, welche Bereiche bei dem Vergleich besonders zu berücksichtigen sind. Diese Berücksichtigung kann beispielsweise über Gewichtungsfaktoren eingeführt werden. Eine Sensitivitätskarte kann prinzipiell auch unter Einbeziehung des Verifikationsdatensatzes bestimmt werden, indem beispielsweise die Änderungen der Planungsdatensätze bezogen auf den Verifikationsdatensatz berücksichtigt werden bzw. einfließen.

Eine Sensitivitätskarte kann beispielsweise erstellt werden, indem der Mittelwert der absoluten HU-Differenzen (HU für Hounsfield Units) zwischen den Planungsdatensätzen pro Voxel berechnet werden. Die Größe dieses Mittelwerts kennzeichnet dann die zu erwartenden Veränderungen, die zwischen den verschiedenen Datensätzen auftreten. Es können auch andere Parameter verwendet werden, um die Sensitivitätskarte zu erstellen, beispielsweise können bekannte statistische Parameter wie das Minimum oder das Maximum der Differenzen, die Standardabweichung usw. verwendet werden.

Die Sensitivitätskarte kann beispielsweise verwendet werden, um eine neue Maske zu erstellen, die diejenigen Bereiche kennzeichnet, die zur Beurteilung der Ähnlichkeit verwendet werden sollen. Die Sensitivitätskarte kann auch dazu verwendet werden, unterschiedliche Regionen innerhalb des Bereichs, bezüglich dessen die Ähnlichkeit beurteilt wird, verschieden stark zu gewichten, um beispielsweise empfindliche Bereiche stärker zu berücksichtigen. Sensitivitätskarten können auch nur für bestimmte Teilbereiche der Datensätze bestimmt werden.

In einer vorteilhaften Ausführungsform erfolgt die Erstellung der Bestrahlungspläne unter gleichen Vorgaben, insbesondere was die zu verwendenden Sicherheitssäume betrifft. Dies bedeutet, dass bei der Erstellung der Bestrahlungspläne dieselbe Bestrahlungsstrategie verwendet wird. Lediglich die unterschiedliche Lage, d.h. die unterschiedliche Position und/oder Form des Zielvolumens wird dann bei der Erstellung der Bestrahlungspläne berücksichtigt. Weitere Vorgaben, wie Solldosis, zu schonende Strukturen, Sicherheitssäume etc. bleiben gleich. Hierdurch sind die Bestrahlungspläne prinzipiell miteinander vergleichbar. Dies vereinfacht die Automatisierbarkeit des Verfahrens, da die Bestrahlungsstrategie nicht geändert wird.

Die erfindungsgemäße Vorrichtung zur Auswahl eines Bestrahlungsdatensatzes ist ausgebildet zur Durchführung eines der oben beschriebenen erfindungsgemäßen Verfahren und umfasst insbesondere:

- eine Eingabeeinrichtung, mit welcher ein Verifikationsdatensatz und mehrere Planungsdatensätze ladbar sind,
- eine Vergleichseinrichtung, mit welcher ein Vergleich des Verifikationsdatensatzes mit den mehreren Planungsdatensätzen bezüglich einer Ähnlichkeit durchführbar ist,
- eine Auswertungseinrichtung, mit welcher ein Planungsdatensatz aus den mehreren Planungsdatensätzen bestimmbar ist, welcher die größte Ähnlichkeit mit dem Verifikationsdatensatz aufweist, und
- eine Auswahleinrichtung, mit welcher ein Bestrahlungsplan, der dem durch die Auswertungseinrichtung bestimmten Planungsdatensatz zugeordnet ist, ladbar ist.

Die einzelnen Untereinheiten der Vorrichtung können dabei vorzugsweise in einer einzigen Rechnereinheit realisiert sein, die so ausgebildet ist, dass die Rechnereinheit die Aufgabe der einzelnen Untereinheiten ausführen kann.

Die Rechnereinheit kann beispielsweise eine Steuerungseinheit einer Bestrahlungsanlage, z.B. eine Partikeltherapieanlage sein, der z.B. mit einer Bestrahlungsplanungsseinrichtung in Verbindung stehen, welche zur Erstellung der Bestrahlungspläne für die Planungsdatensätzen ausgebildet ist.

Erfindungsgemäß ist ein Bereich in einem der Planungsdatensätze und/oder in dem Verifikationsdatensatz bestimmbar, derart, dass der Vergleichs des Verifikationsdatensatzes mit den mehreren Planungsdatensätzen hinsichtlich der Ähnlichkeit lediglich in dem bestimmten Bereich durchgeführt wird.

Die Vergleichseinrichtung kann weiterhin derart ausgebildet sein, bei dem Vergleich der mehreren Planungsdatensätze mit dem Verifikationsdatensatz eine Sensitivitätskarte zu verwenden, welche eine Variabilität zwischen den mehreren Planungsdatensätzen bezüglich unterschiedlicher Regionen charakterisiert.

Die Sensitivitätskarte kann beispielsweise ermittelt werden, wenn die Planungsdatensätzen untereinander und/oder mit dem Verifikationsdatensatz verglichen werden.

Eine erfindungsgemäße Bestrahlungsanlage, insbesondere eine Partikeltherapieanlage, weist eine derartige Vorrichtung auf.

Ausführungsformen der Erfindung mit vorteilhaften Weiterbildungen gemäß den Merkmalen der abhängigen Ansprüche werden anhand der folgenden Zeichnung näher erläutert, ohne jedoch darauf beschränkt zu sein. Es zeigen:

Fig. 1    einen schematischen Überblick über eine Partikeltherapieanlage,

Fig. 2    eine schematische Darstellung einer Ausführungsform der erfindungsgemäßen Vorrichtung,

Fig. 3    ein Schema, welches den Vergleich eines Verifikationsdatensatzes mit mehreren Planungsdatensätzen illustriert,

Fig. 4    ein Schema, welches den Vergleich eines Verifikationsdatensatzes mit mehreren Planungsdatensätzen illustriert, wobei die Datensätze eine zeitliche Dimension umfassen,

Fig. 5    verschiedene Bereiche an einem Datensatz, in welchen eine Beurteilung der Ähnlichkeit durchgeführt wird,

Fig. 6    eine schematische Darstellung einer Sensitivitätskarte,

Fig. 7    ein Diagramm, welches die Korrelation zwischen dem Ähnlichkeitsmaß und der Qualität des ausgewählten Bestrahlungsplans angibt.

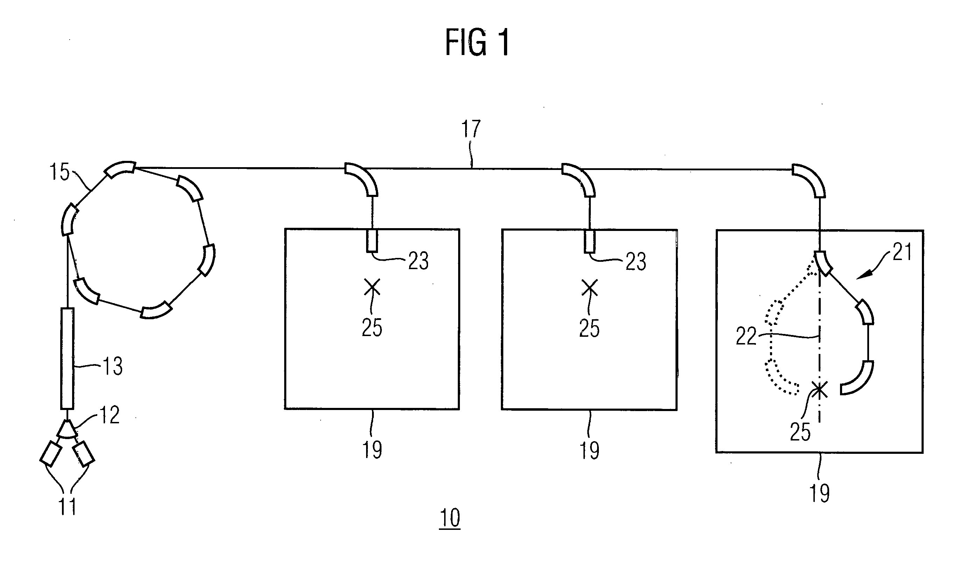

Fig. 1 zeigt einen schematischen Überblick über den Aufbau einer Partikeltherapieanlage 10. In einer Partikeltherapieanlage 10 erfolgt insbesondere eine Bestrahlung eines Körpers, insbesondere eines tumorerkrankten Gewebes, oder eines Phantoms mit einem Partikelstrahl.

Als Partikel werden vornehmlich Ionen wie beispielsweise Protonen, Heliumionen, Kohlenstoffionen oder andere Partikel wie Pionen eingesetzt. Üblicherweise werden derartige Partikel in einer Partikelquelle 11 erzeugt. Wenn, wie in Figur 1 dargestellt, zwei Partikelquellen 11 vorhanden sind, die zwei verschiedene Ionensorten erzeugen, kann zwischen diesen beiden Ionensorten innerhalb eines kurzen Zeitintervalls umgeschaltet werden. Dazu wird beispielsweise ein Schaltmagnet 12 verwendet, der zwischen den Ionenquellen 11 einerseits und einem Vorbeschleuniger 13 andererseits angeordnet ist. Z.B. kann hierdurch die Partikeltherapieanlage 10 mit Protonen und mit Kohlenstoffionen gleichzeitig betrieben werden.

Die von der oder einer der Ionenquellen 11 erzeugten und gegebenenfalls mit dem Schaltmagneten 12 ausgewählten Ionen werden in dem Vorbeschleuniger 13 auf ein erstes Energieniveau beschleunigt. Der Vorbeschleuniger 13 ist beispielsweise ein Linearbeschleuniger (LINAC für engl.: "LINear ACcelerator"). Anschließend werden die Partikel in einen Beschleuniger 15, beispielsweise ein Synchrotron oder Zyklotron, eingespeist. In dem Beschleuniger 15 werden sie auf hohe Energien, wie sie zur Bestrahlung nötig sind, beschleunigt. Nachdem die Partikel den Beschleuniger 15 verlassen, führt ein Hochenergiestrahl-Transportsystem 17 den Partikelstrahl zu einem oder mehreren Bestrahlungsräumen 19. In einem Bestrahlungsraum 19 werden die beschleunigten Partikel auf einen zu bestrahlenden Körper gerichtet. Je nach Ausgestaltung erfolgt dies von einer festen Richtung (in so genannten "fixed beam"-Räumen) aus oder aber über eine um eine Achse 22 bewegliche rotierbare Gantry 21 von verschiedenen Richtungen aus.

Im Bestrahlungsraum 19 tritt der Partikelstrahl aus einem Strahlauslass 23 aus und trifft auf ein zu bestrahlendes Zielvolumen, das sich üblicherweise im Isozentrum 25 eines Bestrahlungsraums befindet.

Der anhand der Figur 1 dargestellte Grundaufbau einer Partikeltherapieanlage 10 ist beispielhaft für Partikeltherapieanlagen, kann aber auch hiervon abweichen.

Die nachfolgend beschriebenen Ausführungsbeispiele können sowohl im Zusammenhang mit der anhand von Fig. 1 dargestellten Partikeltherapieanlage 10 als auch mit anderen Bestrahlungsanlagen eingesetzt werden, bei denen ein Bestrahlungsplan ausgewählt werden soll.

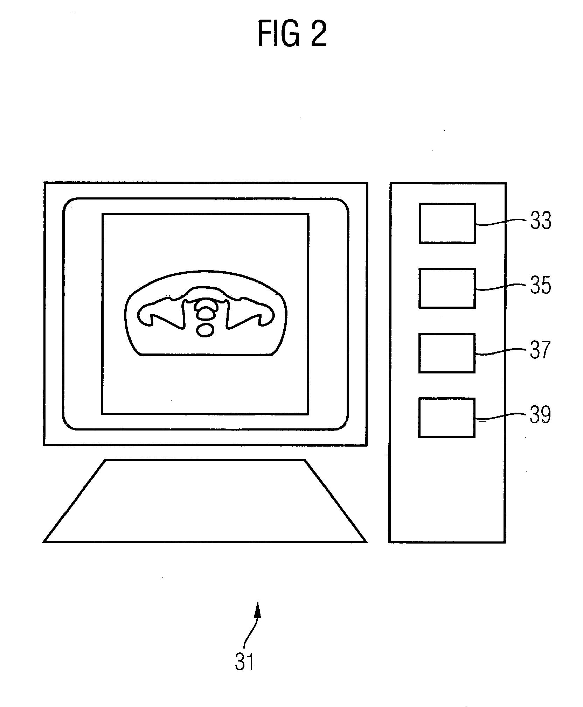

Fig. 2 zeigt eine Vorrichtung 31, welche zur Auswahl eines Bestrahlungsplans ausgebildet ist.

Die Vorrichtung 31 kann beispielsweise in einer Rechnereinheit, welche für die Bestrahlungsplanung oder die Bestrahlungsdurchführung eingesetzt wird, implementiert werden.

Die Vorrichtung 31 umfasst eine Eingabeeinrichtung 33, mit welcher ein Verifikationsdatensatz und mehrere zuvor erstellte Planungsdatensätze ladbar sind. Derartige Datensätze sind üblicherweise in einer Datenbank gespeichert, so dass die Eingabeeinrichtung 33 Schnittstellen aufweist, über die die Datensätze in die Vorrichtung 31 aufgenommen werden können.

Weiterhin umfasst die Vorrichtung 31 eine Vergleichseinrichtung 35, mit welcher ein Ähnlichkeitsmaß ausgewählt oder geladen werden kann und ein Vergleich des Verifikationsdatensatzes mit den mehreren Planungsdatensätzen bezüglich einer Ähnlichkeit durchgeführt werden kann.

Die Vorrichtung 31 umfasst eine Auswertungseinrichtung 37, mit welcher ein Planungsdatensatz aus den mehreren Planungsdatensätzen bestimmt wird, welcher die größte Ähnlichkeit mit dem Verifikationsdatensatz aufweist.

Die Vorrichtung 31 umfasst eine Auswahleinrichtung 39, mit welcher ein Bestrahlungsplan, der dem durch die Auswertungseinrichtung bestimmten Planungsdatensatz zugeordnet ist, ladbar ist. Dieser Bestrahlungsplan kann der Bestrahlungsanlage 10 übermittelt werden, sodass eine Bestrahlung gemäß dem Bestrahlungsplan durchgeführt wird.

Die hier beschriebenen einzelnen Untereinheiten 33, 35, 37, 39 können auch in einer einzigen Rechnereinheit implementiert werden, wobei die Rechnereinheit mittels einer geeigneten Software und/oder Hardware derart ausgebildet ist, dass die Funktionalitäten der einzelnen Untereinheiten durchgeführt werden.

Die Vorrichtung 31 ist dabei derart ausgebildet, dass mit der Vorrichtung 31 die anhand der nachfolgenden Abbildungen beschriebenen Ausführungsbeispiele ausgeführt werden können.

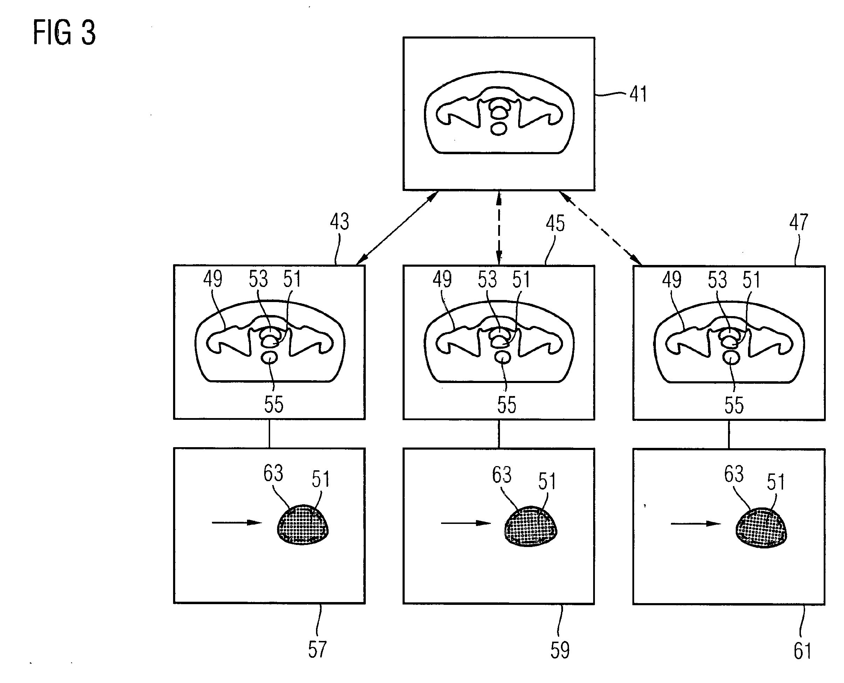

Fig. 3 zeigt schematisch den Vergleich eines Verifikationsdatensatzes 41 mit mehreren Planungsdatensätzen 43, 45, 47.

Im Vorfeld wurden mehrere Planungsdatensätze 43, 45, 47 aufgezeichnet, beispielsweise mit einem CT-Gerät. In den Planungsdatensätzen 43, 45, 47 symbolisch dargestellt ist ein Transversalschnitt durch das Becken. In dem Transversalschnitt deutlich zu erkennen ist eine knöcherne Struktur 49, welche in den drei Planungsdatensätzen 43, 45, 47 stets die gleiche Form und Lage aufweist. Das zu bestrahlende Zielvolumen ist die Prostata 51, welche von der Blase 53 und von dem Rektum 55 flankiert wird. Aufgrund des unterschiedlichen Füllungszustandes der Blase 53 und der unterschiedlichen Lage und Form des Rektums 55 weist die Prostata 51 in den drei Planungsdatensätzen 43, 45, 47, welche an unterschiedlichen Zeitpunkten aufgezeichnet worden sind, eine geringfügig unterschiedliche Lage und Form auf. Für jede dieser drei Planungsdatensätze 43, 45, 47 wurde im Vorfeld ein Bestrahlungsplan 57, 59, 61 erstellt, indem festgelegt wird, wie das Zielvolumen, d.h. die Prostata 51, zu bestrahlen ist.

In den drei Bestrahlungsplänen 57, 59, 61 wurde das klinische Zielvolumen um die Prostata 51 dabei jeweils um einen gleich großen Sicherheitssaum 63 erweitert. Basierend hierauf wurde eine Vielzahl von Rasterpunkte festgelegt, die sukzessive mit einem Partikelstrahl abgescannt werden sollen. Der Pfeil in den Bestrahlungsplänen 57, 59, 61 kennzeichnet dabei eine der Einstrahlrichtungen des Partikelstrahls.

Im Vorfeld einer geplanten Bestrahlung wird ein Verifikationsdatensatzes 41 aufgezeichnet.

Um einen geeigneten Bestrahlungsplan aus den Bestrahlungsplänen 57, 59, 61 auszuwählen, erfolgt ein Vergleich des Verifikationsdatensatzes 41 mit den drei Planungsdatensätzen 43, 45, 47. Der Vergleich richtet sich auf eine Ähnlichkeit zwischen dem Verifikationsdatensatzes 41 und den Planungsdatensätzen 43, 45, 47. In diesem dargestellten Fall weist der linke Planungsdatensatz 43 die größte Ähnlichkeit mit dem Verifikationsdatensatzes 41 hinsichtlich der Lage und der Form der Prostata 51 und der die Prostata umgebenden Organe 53, 55 auf.

Der diesem Planungsdatensatz 43 zu Grunde liegende Bestrahlungsplan 57 kann dann - falls eine nachfolgende Bestrahlung stattfindet - der Bestrahlung zugrunde gelegt werden.

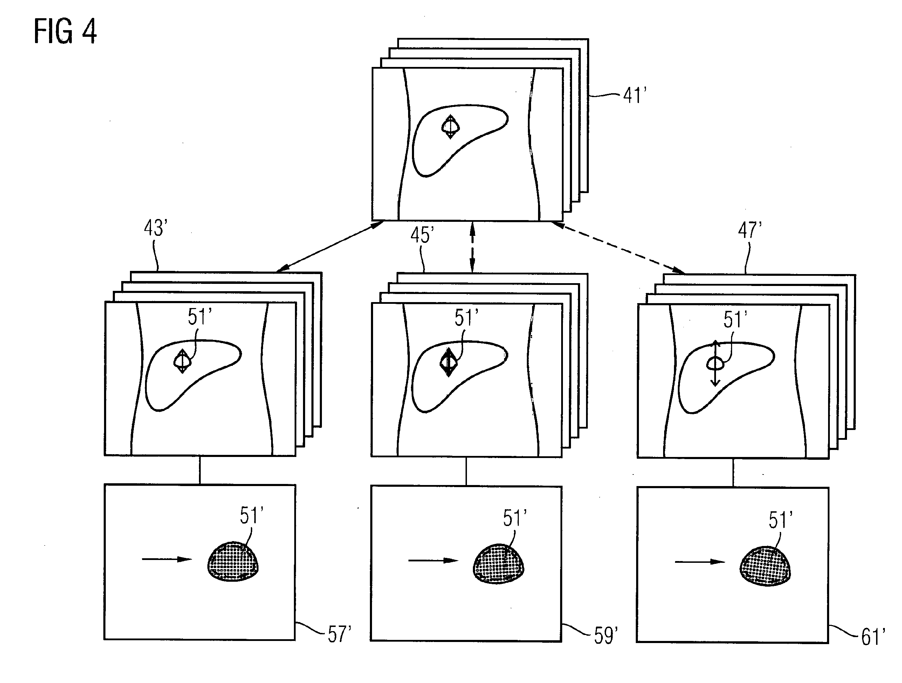

Fig. 4 illustriert ein ähnliches Verfahren, diesmal nur für ein Zielvolumen 51', welches sich während einer Bestrahlungssitzung derart stark bewegt, dass die Bewegung bei der Planung der Bestrahlung berücksichtigt werden muss.

Ein derartiger Fall tritt beispielsweise bei Tumoren auf, welche durch eine Atembewegung bewegt werden, wie beispielsweise bei einer Lebermetastase 51'.

Die Bewegung lässt sich dadurch erfassen, dass die Planungsdatensätze 43', 45', 47' und/oder der Verifikationsdatensatz 41' eine zeitliche Dimension umfassen. Dies bedeutet, dass aus einem der Planungsdatensätze 43', 45', 47' bzw. aus dem Verifikationsdatensatz 41' die Bewegung des Objektes ermittelbar ist. Beispielsweise können als Planungsdatensatz und als Verifikationsdatensatz ein 4D-CT aufgezeichnet werden.

Dargestellt sind drei vierdimensionale Planungsdatensätze 43', 45', 47'. Die unterschiedliche Bewegung des Zielvolumens 51', z.B. die Amplitude und Frequenz der Bewegung, ist in der Abbildung durch unterschiedlich lange Pfeile mit unterschiedlicher Dicke symbolisiert.

Auch hier wird wiederum ein vierdimensionaler Verifikationsdatensatz 41' mit den drei vierdimensionalen Planungsdatensätzen 43', 45', 47' verglichen und derjenige Planungsdatensatz 43' identifiziert, der die größte Ähnlichkeit mit dem Verifikationsdatensatz 41' aufweist. Bei dem Vergleich wird diesmal auch die Bewegung des Zielvolumens 51' berücksichtigt. Die anderen Vergleichsmerkmale wie die Position und die Form des Zielvolumens, der Eintrittskanal, die umgebenden Strukturen usw. können weiterhin berücksichtigt werden.

Um die Bewegung zu vergleichen, können z.B. das Zentrum des Zielvolumens 51', die mittlere Position des Zielvolumens 51' und Bewegungsparameter wie beispielsweise die Amplitude und Frequenz miteinander verglichen werden.

Anschließend wird aus den Bestrahlungsplänen 57', 59', 61' derjenige Bestrahlungsplan 57' ausgewählt, der dem Planungsdatensatz 43' zu Grunde liegt, welcher die größte Ähnlichkeit mit dem Verifikationsdatensatz 41' aufweist.

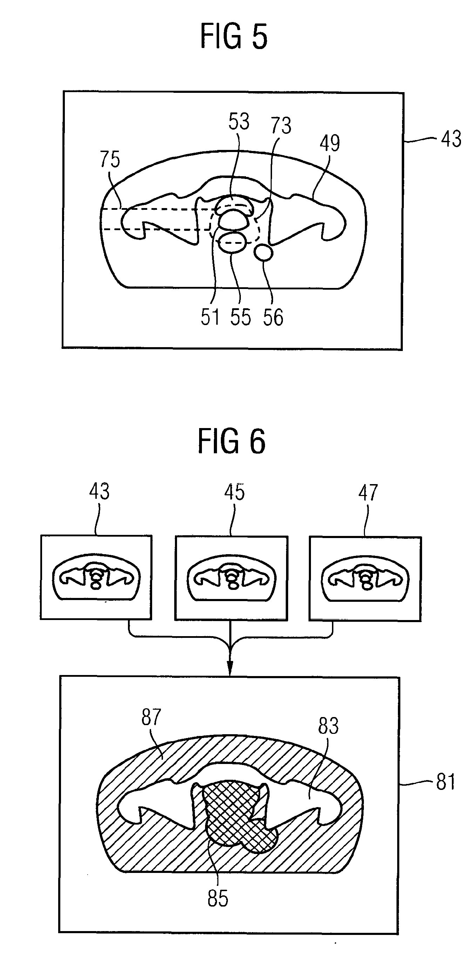

Fig. 5 zeigt einen Planungsdatensatz 43, in welchem bestimmte Regionen markiert sind. Diese Regionen geben an, welche Bereiche des Planungsdatensatzes bzw. des Verifikationsdatensatzes hinsichtlich einer Ähnlichkeit beurteilt werden.

Eine erste Region 73 umfasst das Zielvolumen, in diesem Fall also die Prostata 51. Darüber hinaus umfasst die erste Region 73 die an das Zielvolumen angrenzenden Strukturen bzw. Organe, wie die Blase 53 und das Rektum 55, diese jedoch nur zum Teil. Insbesondere grenzt die erste Region 73 den Bereich, hinsichtlich dessen die Ähnlichkeit beurteilt wird, derart ein, dass weiter entfernt liegende Bereiche nicht in die Beurteilung der Ähnlichkeit einfließen, selbst wenn sie eine hohe Variabilität bezüglich Lage und Form aufweisen. In dem dargestellten Beispiel ist dies beispielsweise ein weiter entfernt liegender Darmabschnitt 56, der jedoch - wenn überhaupt - einen nur sehr geringen Einfluss auf die Lage und auf die Form der Prostata 51 hat. Es hat sich auch gezeigt, dass die umgebende knöcherne Struktur 49 von dem ersten Bereich 73 ausgeschlossen werden soll.

Wenn eine Bestrahlungsplanung mit Partikelstrahlen erfolgt, kann insbesondere ein weiterer Bereich 75, der zumindest teilweise im Eintrittskanal des Partikelstrahls liegt, hinsichtlich einer Ähnlichkeit beurteilt werden. Auf diese Weise kann sichergestellt werden, dass der Partikelstrahl auch eine passende Reichweite hat, da die Reichweite des Partikelstrahls vornehmlich durch Strukturen beeinflusst wird, die in Strahlrichtung vor dem Zielvolumen liegen.

Im Folgenden wird ein Algorithmus beschrieben, der sich besonders bei Bestrahlungsplänen, welche die Prostata 51 betreffen, als günstig erwiesen hat, um einen Bereich auszuwählen, bezüglich welchem die Planungsdatensätze mit dem Verifikationsdatensatz verglichen werden, im folgenden als Vergleichsbereich bezeichnet.

Bei dem Algorithmus wird zunächst das Zielvolumen (CTV für engl: "clinical target volume") bestimmt, in diesem Falle die Prostata 51. Um jedes Voxel, das der Prostata 51 zugeordnet ist, wird ein Quader einer bestimmten Größe gelegt, beispielsweise ein Quader aus 5x5x3 Voxel. Jeder dieser Quader wird dahingehend analysiert, ob in dem Quader wenigstens ein Voxel vorhanden ist, das entweder der Blase 53 oder dem Rektum 55, also den umgebenden Organen, zugeordnet ist.

Weiterhin wird jeder dieser Quader dahingehend analysiert, ob in dem Quader ein Voxel vorhanden ist, das einer knöchernen Struktur 49 wie dem Hüftknochen zugeordnet ist, also eine Struktur, welche von dem Bereich ausgeschlossen werden soll, der der Beurteilung der Ähnlichkeit zu Grunde liegt.

Wenn einer der Quader also ein Voxel enthält, das der Blase 53 oder dem Rektum 55 zugeordnet ist, wird der Vergleichsbereich um diesen Quader erweitert, es sei denn, der Quader enthält zugleich ein Voxel, das einer knöchernen Struktur 49 zugeordnet ist.

Eine Entscheidung, ob letzterer Fall vorliegt, kann bei einem CT-Datensatz anhand von Hounsfield Units (HU) vorgenommen werden. Quader, die ein Voxel der Blase 53 enthalten und ebenfalls ein Voxel über 600 HU, fallen unter diese Kategorie, ebenso wie Quader, die ein Voxel des Rektums 55 enthalten und ebenfalls ein Voxel über 1200 HU. Die Festlegung der Schwellwerte 600 HU bzw. 1200 HU ist dabei willkürlich, hat sich aber bei der Auswertung von CT-Datensätzen, welche die Prostata 51 abbilden, als vorteilhaft erwiesen. Die Schwellwerte können dabei auch abgeändert werden und den jeweils vorliegenden Gegebenheiten angepasst werden.

Der Vergleichsbereich umfasst dann alle Quader, die wie oben beschrieben bestimmt wurden, sowie das Zielvolumen selbst, in diesem Falle also die Prostata 51.

Der eben beschriebene Algorithmus hat den Vorteil, dass er auf einfache Weise weitgehend automatisch implementiert werden kann, so dass eine Interaktion mit einem Anwender in nur sehr geringem Maße notwendig ist, falls überhaupt.

Fig. 6 zeigt schematisch die Erstellung einer Sensitivitätskarte 81, die bei der Auswahl derjenigen Bereiche, die hinsichtlich der Ähnlichkeit beurteilt werden, vorteilhaft eingesetzt werden kann. Die Sensitivitätskarte 81 entsteht aus dem Vergleich von mehreren Planungsdatensätzen 43, 45, 47. Die Sensitivitätskarte 81 gibt an, wie stark die Variabilität einzelner Bereiche zwischen den verschiedenen Planungsdatensätzen 43, 45, 47 ist, vorzugsweise wie groß die Veränderungen von Planungsdatensatz zu Planungsdatensatz pro Voxel sind. In einer anderen bevorzugten Ausführung können auch die Änderungen zwischen Verifikationsdatensatz und Planungsdatensätzen zur Erstellung der Sensitivitätskarte berücksichtigt werden.

Um das zu Grunde liegende Konzept zu erklären, weist die hier dargestellte Sensitivitätskarte 81 der Einfachheit halber lediglich drei diskrete Bereiche 83, 85, 87 auf. Eine Sensitivität bezüglich der Variabilität zwischen den Planungsdatensätzen kann jedoch auch voxelweise bestimmt werden.

Die Sensitivitätskarte zeigt hier in einem Bereich 83 von knöchernen Strukturen eine sehr geringe Variabilität an. Die höchste Variabilität weist ein Bereich 85 um die Prostata bzw. um die Blase und um den Darm auf. Ein anderer Bereich 87, wie beispielsweise umgebendes Fett bzw. Muskelgewebe weisen eine mittlere Variabilität auf.

Die Sensitivitätskarte 81 kann z.B. dazu verwendet werden, bei dem Vergleich zwischen dem Verifikationsdatensatz und den Planungsdatensätzen bestimmte Bereiche unterschiedlich stark zu gewichten, je nachdem, wie stark die zu erwartenden Änderungen in den jeweiligen Bereichen sind.

Im Folgenden wird ein Algorithmus beschrieben, wie bei dem Vergleich von Datensätzen, in welchen die Prostata abgebildet ist, eine Sensitivitätskarte 81 erstellt und vorteilhaft eingesetzt werden kann.

In einem ersten Schritt werden zwischen dem Verifikationsdatensatz 41 und den verschiedenen Planungsdatensätzen 43, 45, 47 Differenzdatensätze erstellt, indem von dem Verifikationsdatensatz jeweils ein Planungsdatensatz voxelweise subtrahiert wird.

Anschließend wird voxelweise der Mittelwert und die Standardabweichung aus den erzeugten Differenzdatensätzen ermittelt und so eine Mittelwert-Sensitivitätskarte bzw. eine Standardabweichung-Sensitivitätskarte erzeugt. Aus diesen statistischen Werten lässt sich entnehmen, wie stark die Planungsdatensätzen 43, 45, 47 untereinander bezüglich der einzelnen Voxel variieren.

Die ermittelte Mittelwert-Sensitivitätskarte und die ermittelte Standardabweichung-Sensitivitätskarte können dann einer Schwellwert-Betrachtung unterzogen werden. Um diejenigen Voxel zu identifizieren, welche eine große Variation zwischen den CT-Datensätzen aufweisen, wird ein unterer Schwellwert für die Standardabweichung-Sensitivitätskarte eingeführt. Als unterer Schwellwert hat sich ein Wert von 20 HU als vorteilhaft erwiesen. Um sich auf Änderungen zu beziehen, die durch Änderungen des Weichteilgewebes bedingt sind und nicht durch rektale Gasansammlungen, wurde ein oberer Schwellwert für die Mittelwert-Sensitivitätskarte eingeführt. Als oberer Schwellwert hat sich ein Wert von 50 HU als vorteilhaft erwiesen.

Anschließend kann der Vergleichsbereich, wie er weiter oben beschrieben worden ist, weiter verfeinert werden, indem der verbesserte Vergleichsbereich nur diejenigen Voxel umfasst, die oberhalb des unteren Schwellwertes der Standardabweichung-Sensitivitätskarte und unterhalb des oberen Schwellwertes der Mittelwert-Sensitivitätskarte liegen.

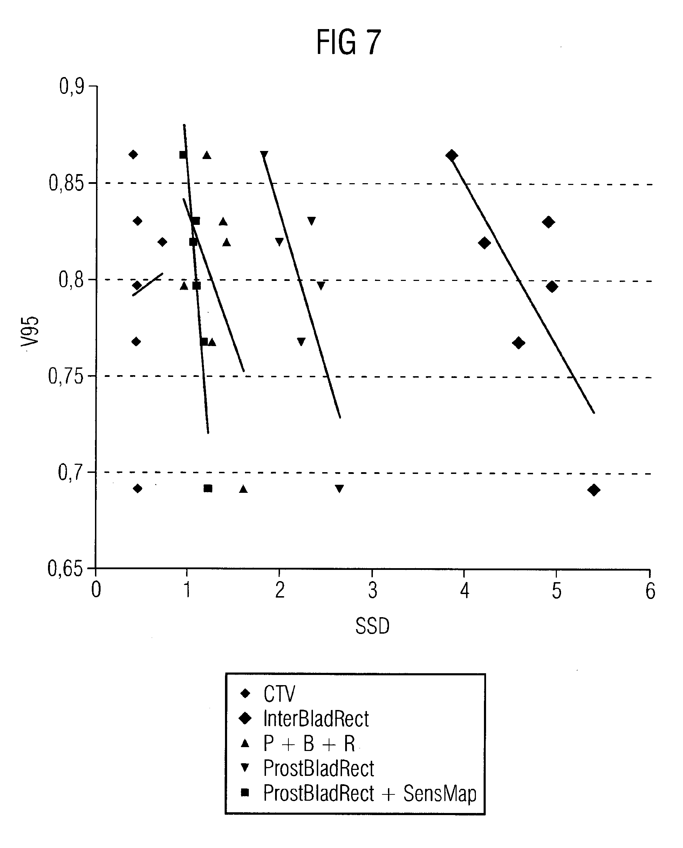

Fig. 7 zeigt den Zusammenhang zwischen der Summe der quadratischen Abweichungen in dem Vergleichsbereich und der Dosisbelegung des Zielvolumens, und zwar für verschiedene Arten von Vergleichsbereichen.

Die Summe der quadratischen Abweichungen (SSD für engl: "sum of squared differences") errechnet sich für einen Vergleichsbereich anhand folgender Formel:

$$SSD = \sum_m \left(D_m - P_m\right)^2 \Big/ NV \; ,$$

wobei m der Index der Voxel des Vergleichsbereich, $D_m$ und $P_m$ die Werte eines Voxels m im Verifikationsdatensatz bzw. im Planungsdatensatz und NV die Anzahl aller Voxel im Vergleichsbereich bezeichnet.

Die SSD gibt folglich an, wie groß der Unterschied zwischen einem Verifikationsdatensatz 41 und einem Planungsdatensatz 43, 45, 47 in dem Vergleichsbereich ist, und ist damit ein Maß für die Ähnlichkeit zwischen einem Planungsdatensatz 43, 45, 47 und dem Verifikationsdatensatz 41.

Der SSD (also dem Wert der x-Achse) steht der V95-Wert gegenüber, ein Index, der angibt, wie gut das Zielvolumen mit einer Dosis überdeckt wird (engl: "traget coverage"), gegenübergestellt. Der V95-Wert ist ein in der Strahlentherapie gängiger Wert und gibt an, welcher Anteil von dem Zielvolumen mit mindestens 95% der Solldosis belegt wird, wenn ein bestimmter Bestrahlungsplan angewendet wird.

Zwischen der SSD und dem V95-Wert gibt es einen Zusammenhang. Der Zusammenhang hängt davon ab, welcher Vergleichsbereich bei der Ermittlung der SSD zugrunde gelegt wurde.

Wenn bei der Ermittlung der SSD ein Vergleichsbereich zugrunde gelegt wird, wie er weiter oben beschrieben ist - Prostata 51 mit angrenzenden Strukturen wie der angrenzende Teil der Blase 53 und der angrenzende Teil des Rektums 55, verbessert mithilfe der Sensitivitätskarte 81 - im Diagramm als "ProstBladRect + SensMap" bezeichnet -, herrscht eine gute Korrelation zwischen SSD und dem V95-Wert.

Dies belegt, dass es möglich ist, einen passenden Bestrahlungsplan 57, 59, 61 auszuwählen, indem lediglich ein Vergleich des Verifikationsdatensatzes 41 mit verschiedenen Planungsdatensätzen 43, 45, 47 bezüglich einer Ähnlichkeit durchgeführt wird. Für die Bestrahlungsplanung der Prostata 51 hat sich gezeigt, dass sich die beste Korrelation ergibt, wenn dieser Vergleichsbereich dem Vergleich von Planungsdatensatz 43, 45, 47 zu Verifikationsdatensatz 41 zu Grunde gelegt wird und das Ähnlichkeitsmaß über eine SSD ermittelt wird.

Ebenfalls gute Korrelationen, wenngleich mit einer etwas schlechteren Korrelation, ergeben sich, wenn als Vergleichsbereiche zu Grunde gelegt werden:

- die Prostata 51 mit dem angrenzenden Teil der Blase 53 und des Rektums 55, diesmal jedoch ohne Verbesserung durch eine Sensitivitätskarte, im Diagramm als "ProstBladRect" bezeichnet,

- die Prostata 51 und die gesamte Blase 53 und das gesamte Rektum 55, im Diagramm als "P + B + R" bezeichnet,

- lediglich die Schnittstellen Prostata/Blase und Prostata /Rektum, im Diagramm als "InterBladRect" bezeichnet.

Die schlechteste Korrelation ergibt sich, wenn als Vergleichsbereich lediglich die Prostata 51 zugrunde gelegt wird, im Diagramm als "CTV" bezeichnet.

Eine andere Möglichkeit, das Ähnlichkeitsmaß zu bestimmen, ist ein Korrelationskoeffizient, der sich nachfolgender Formel errechnet:

$$CC = \frac{\sum_m \left(D_m - \overline{D}\right)\left(P_m - \overline{P}\right)}{\sqrt{\left(\sum_m \left(D_m - \overline{D}\right)^2\right)\left(\sum_m \left(P_m - \overline{P}\right)^2\right)}}$$

wobei m der Index der Voxel des Vergleichsbereich ist, $D_m$ und $P_m$ die Werte eines Voxels m im Verifikationsdatensatz bzw. im Planungsdatensatz und $\overline{D}$ bzw. $\overline{P}$ die Mittelwerte der Voxel im Vergleichsbereich sind.

Für die Bestrahlungsplanung für die Prostata 51 hat sich aber gezeigt, dass der Korrelationskoeffizient schlechtere Ergebnisse liefert als die SSD.

Dies kann jedoch für andere Bestrahlungsszenarien, z.B. andere Tumore oder andere Organe, anders sein.

Anhand der in Fig. 5 dargestellten Korrelationskurven kann auf einfache Weise getestet werden, ob ein Vergleichsbereich oder eine Vorschrift, nach der ein Ähnlichkeitsmaß zwischen dem Verifikationsdatensatz und einem der Planungsdatensätzen ermittelt wird, sich dafür eignen, einen Bestrahlungsplan auszuwählen.

Hierzu muss lediglich die Korrelation zwischen dem Ähnlichkeitsmaß und dem V95-Wert getestet werden. Sobald sich herausstellt, dass die Korrelation gut genug ist, kann das Ähnlichkeitsmaß und/oder der Vergleichsbereich im Verfahren zur Ermittlung des Bestrahlungsplans 57, 59, 61 verwendet werden.

Bezugszeichenliste

| 10 | Bestrahlungsanlage |
|---|---|
| 11 | Ionenquelle |
| 12 | Schaltmagnet |
| 13 | Vorbeschleuniger |
| 15 | Beschleuniger |
| 17 | Hochenergiestrahl-Transportsystem |
| 19 | Bestrahlungsraum |
| 21 | Gantry |
| 22 | Achse |
| 23 | Strahlauslass |
| 25 | Isozentrum |

| 31 | Vorrichtung |
|---|---|
| 33 | Eingabeeinrichtung |
| 35 | Vergleichseinrichtung |
| 37 | Auswertungsvorrichtung |
| 39 | Auswahleinrichtung |

| 41, 41' | Verifikationsdatensatz |
|---|---|
| 43, 45, 47, 43', 45', 47' | Planungsdatensatz |
| 49 | knöcherne Struktur |
| 51, 51' | Zielvolumen |
| 53 | Blase |
| 55 | Rektum |

57, 59, 61, 57', 59', 61'    Bestrahlungsplan
63                           Sicherheitssaum

73    Bereich
75    weiterer Bereich

81    Sensitivitätskarte
83, 85, 87    Bereich

**Patentansprüche**

1. Verfahren zur Bestimmung und zur Auswahl eines Bestrahlungsplans (57, 59, 61; 57', 59', 61') für die Strahlentherapie an einem Patienten, aufweisend folgende Schritte:

   in einer ersten Phase im Vorfeld einer geplanten Bestrahlung:

   - Erfassung von mehreren Planungsdatensätzen (43, 45, 47; 43', 45', 47'), in welchen ein zu bestrahlendes Zielvolumen (51, 51') mit unterschiedlicher Lage in einem Zielobjekt abgebildet ist, und
   - Erstellung eines Bestrahlungsplans (57, 59, 61) für jeden dieser Planungsdatensätze (41, 43, 45; 41', 43', 45'),

   in einer der ersten Phase nachfolgenden zweiten Phase im Vorfeld einer geplanten Bestrahlung:

   - Aufzeichnen eines Verifikationsdatensatzes (41, 41'),
   - Vergleichen des Verifikationsdatensatzes (41, 41') mit den mehreren Planungsdatensätzen (43, 45, 47; 43', 45', 47') hinsichtlich einer Ähnlichkeit,
   - Auswählen eines Planungsdatensatzes (43, 43') aus den mehreren Planungsdatensätzen (43, 45, 47; 43', 45', 47'), welcher die größte Ähnlichkeit mit dem Verifikationsdatensatz (41, 41') aufweist,
   - Auswählen des Bestrahlungsplans (57, 57'), der dem ausgewählten Planungsdatensatz (43, 43') zugeordnet ist, und Laden dieses Bestrahlungsplans mit einer Auswahleinrichtung,

   **dadurch gekennzeichnet, dass**
   ein Bereich (73, 75) in einem der Planungsdatensätze (43, 45, 47; 43', 45', 47') und/oder in dem Verifikationsdatensatz (41, 41') ausgewählt wird, und wobei der Verifikationsdatensatz (41, 41') mit den mehreren Planungsdatensätzen (43, 45, 47; 43', 45', 47') lediglich in diesem Bereich (73, 75) hinsichtlich der Ähnlichkeit verglichen wird,
   dass der Bereich (73, 75) das Zielvolumen (51, 51') und zumindest einen an das Zielvolumen (51, 51') angrenzenden Bereich (53, 55) umfasst, und
   dass der Bereich (73, 75) zumindest einen Teilbereich (49, 56) in einem der Planungsdatensätze (43, 45, 47; 43', 45', 47') und/oder in dem Verifikationsdatensatz (41, 41') nicht umfasst, welcher Teilbereich (49, 56) keinen Berührungspunkt mit dem Zielvolumen (41, 41') hat.

2. Verfahren nach Anspruch 1, wobei der Bereich einen Eintrittskanal (75) eines Behandlungsstrahls umfasst.

3. Verfahren nach einem der Ansprüche 1 bis 2, wobei der Bereich (73, 75) zumindest eine an das Zielvolumen (51, 51') angrenzende Struktur (53, 55) lediglich teilweise umfasst.

4. Verfahren nach einem der Ansprüche 1 bis 3, wobei die mehreren Planungsdatensätzen (43', 45', 47') und/oder der Verifikationsdatensatz (41') eine zeitliche Dimension umfassen.

5. Verfahren nach einem der Ansprüche 1 bis 4, wobei bei dem Vergleich der mehreren Planungsdatensätze (43, 45, 47; 43', 45', 47') mit dem Verifikationsdatensatz (41, 41') eine Sensitivitätskarte (81) verwendet wird, welche eine Variabilität zwischen den mehreren Planungsdatensätzen (43, 45, 47; 43', 45', 47') bezüglich unterschiedlicher Regionen (83, 85, 87) charakterisiert.

6. Verfahren nach einem der Ansprüche 1 bis 5, wobei die Erstellung der Bestrahlungspläne bei den Planungsdatensätzen (43, 45, 47; 43', 45', 47') unter gleichen Vorgaben, insbesondere bezüglich der zu verwendenden Sicherheitssäume (63), erfolgt.

7. Vorrichtung zur Bestimmung und zur Auswahl eines Bestrahlungsplans (57, 59, 61; 57', 59', 61') für die Strahlentherapie an einem Patienten, aufweisend:

eine Eingabeeinrichtung (33), mit welcher ein Verifikationsdatensatz (41, 41') und mehrere Planungsdatensätze (43, 45, 47; 43', 45', 47') ladbar sind,
eine Vergleichseinrichtung (35), mit welcher im Vorfeld einer geplanten Bestrahlung ein Vergleich des Verifikationsdatensatzes (41, 41') mit den mehreren Planungsdatensätzen (43, 45, 47; 43', 45', 47') bezüglich einer Ähnlichkeit durchführbar ist,
eine Auswertungseinrichtung (37), mit welcher im Vorfeld der geplanten Bestrahlung ein Planungsdatensatz (43, 43') aus den mehreren Planungsdatensätzen (43, 45, 47; 43', 45', 47') bestimmbar ist, welcher die größte Ähnlichkeit mit dem Verifikationsdatensatz (41, 41') aufweist, und
eine Auswahleinrichtung (39), mit welcher im Vorfeld der geplanten Bestrahlung ein Bestrahlungsplan (57, 57'), der dem durch die Auswertungseinrichtung (37) bestimmten Planungsdatensatz (41, 41') zugeordnet ist, ladbar ist,
**dadurch gekennzeichnet, dass**
zusätzlich die Vergleichseinrichtung (35) derart ausgebildet ist, dass ein Bereich (73, 75) in einem der Planungsdatensätze (43, 45, 47; 43', 45', 47') und/oder in dem Verifikationsdatensatz (41, 41') bestimmbar ist, wobei der Vergleich des Verifikationsdatensatzes (41, 41') mit den mehreren Planungsdatensätzen (43, 45, 47; 43', 45', 47') hinsichtlich der Ähnlichkeit lediglich in dem Bereich (73, 75) stattfindet,
dass der Bereich (73, 75) das Zielvolumen (51, 51') und zumindest einen an das Zielvolumen (51, 51') angrenzenden Bereich (53, 55) umfasst, und
dass der Bereich (73, 75) zumindest einen Teilbereich (49, 56) in einem der Planungsdatensätze (43, 45, 47; 43', 45', 47') und/oder in dem Verifikationsdatensatz (41, 41') nicht umfasst, welcher Teilbereich (49, 56) keinen Berührungspunkt mit dem Zielvolumen (41, 41') hat.

8. Vorrichtung nach Anspruch 7, wobei der Bereich einen Eintrittskanal (75) eines Behandlungsstrahls umfasst.

9. Vorrichtung nach Anspruch 7 oder 8, wobei
die Vergleichseinrichtung (35) ausgebildet ist, bei dem Vergleich der mehreren Planungsdatensätze (43, 45, 47; 43', 45', 47') mit dem Verifikationsdatensatz (41, 41') eine Sensitivitätskarte (83) zu verwenden, welche eine Variabilität zwischen den mehreren Planungsdatensätzen (43, 45, 47; 43', 45', 47') bezüglich unterschiedlicher Regionen (83, 85, 87) charakterisiert.

10. Vorrichtung nach einem der Ansprüche 7 bis 9, wobei zusätzlich eine Bestrahlungsplanungseinrichtung (31) vorgesehen ist, mit welcher für die Planungsdatensätze (43, 45, 47; 43', 45', 47') jeweils ein Bestrahlungsplan (57, 59, 61; 57', 59', 61') erstellbar ist.

11. Bestrahlungsanlage (10) mit einer Vorrichtung nach einem der Ansprüche 7 bis 10.


**Claims**

1. Method for determining and selecting an irradiation plan (57, 59, 61; 57', 59', 61') for radiation therapy on a patient, comprising the following steps:

in a first phase in the run-up to a planned irradiation:

- detecting a plurality of planning data records (43, 45, 47; 43', 45' 47') in which a target volume (51, 51') for irradiating and with varying position is represented in a target object, and
- creating an irradiation plan (57, 59, 61) for each of these planning data records (41, 43, 45; 41', 43' 45'),

in a second phase following the first phase in the run-up to a planned irradiation:

- recording a verification data record (41, 41'),
- comparing the verification data record (41, 41') with the plurality of planning data records (43, 45, 47; 43', 45' 47') with respect to similarity,

- selecting a planning data record (43, 43') from the plurality of planning data records (43, 45, 47; 43', 45' 47') which has the greatest similarity to the verification data record (41, 41'),
- selecting the irradiation plan (57, 57') which is associated with the selected planning data record (43, 43') and loading this irradiation plan with a selector,

**characterised in that**
an area (73, 75) in one of the planning data records (43, 45, 47; 43', 45' 47') and/or in the verification data record (41, 41') is selected and wherein the verification data record (41, 41') is only compared in this area (73, 75) with the plurality of planning data records (43, 45, 47; 43', 45' 47') with respect to similarity,
that the area (73, 75) includes the target volume (51, 51') and at least one area (53, 55) adjoining the target volume (51, 51') and
that the area (73, 75) does not include at least one portion (49, 56) in one of the planning data records (43, 45, 47; 43', 45' 47') and/or the verification data record (41, 41'), which portion (49, 56) does not have a point of contact with the target volume (41, 41').

2.  Method according to claim 1, wherein the area includes an entry channel (75) of a treatment beam.

3.  Method according to one of claims 1 to 2, wherein the area (73, 75) only partially includes at least one structure (53, 55) adjoining the target volume (51, 51').

4.  Method according to one of claims 1 to 3, wherein the plurality of planning data records (43', 45', 47') and/or the verification data record (41') include a time dimension.

5.  Method according to one of claims 1 to 4, wherein a sensitivity map (81) is used when comparing the plurality of planning data records (43, 45, 47; 43', 45' 47') with the verification data record (41, 41') and this characterises a variability between the plurality of planning data records (43, 45, 47; 43', 45' 47') with respect to different regions (83, 85, 87).

6.  Method according to any one of claims 1 to 5, wherein the irradiation plans for the planning data records (43, 45, 47; 43', 45' 47') are created under identical specifications, in particular with respect to the safety margins (63) to be used.

7.  Device for determining and selecting an irradiation plan (57, 59, 61; 57', 59', 61') for radiation therapy on a patient, comprising:

    an input mechanism (33) with which a verification data record (41, 41') and a plurality of planning data records (43, 45, 47; 43', 45' 47') can be loaded,
    a comparator (35) with which, in the run-up to a planned irradiation, the verification data record (41, 41') can be compared with the plurality of planning data records (43, 45, 47; 43', 45' 47') with respect to a similarity,
    an evaluation mechanism (37) with which, in the run-up to the planned irradiation, a planning data record (43, 43'), which has the greatest similarity to the verification data record (41, 41'), can be determined from the plurality of planning data records (43, 45, 47; 43', 45' 47'), and
    a selector (39) with which, in the run-up to the planned irradiation, an irradiation plan (57, 57'), which is associated with the planning data record (41, 41') determined by the evaluation mechanism (37), can be loaded
    **characterised in that**
    the comparator (35) is also designed in such a way that an area (73, 75) can be determined in one of the planning data records (43, 45, 47; 43', 45' 47') and/or in the verification data record (41, 41'), wherein the verification data record (41, 41') is only compared in the area (73, 75) with the plurality of planning data records (43, 45, 47; 43', 45' 47') with respect to similarity.
    that the area (73, 75) includes the target volume (51, 51') and at least one area (53, 55) adjoining the target volume (51, 51') and
    that the area (73, 75) does not include at least one portion (49, 56) in one of the planning data records (43, 45, 47; 43', 45' 47') and/or in the verification data record (41, 41'), which portion (49, 56) does not have a point of contact with the target volume (41, 41').

8.  Device according to claim 7, wherein the area includes an entry channel (75) of a treatment beam.

9.  Device according to claim 7 or 8, wherein, when comparing the plurality of planning data records (43, 45, 47; 43', 45' 47') with the verification data record (41, 41'), the comparator (35) is designed to use a sensitivity map (83) which characterises a variability between the plurality of planning data records(43, 45, 47; 43', 45' 47') with respect to different regions (83, 85, 87).

10. Device according to any one of claims 7 to 9, wherein an irradiation planning device (31) is also provided with which a respective irradiation plan (57, 59, 61; 57'. 59' 61') can be created for the planning data records (43, 45, 47; 43', 45' 47').

11. Irradiation facility (10) having a device according to one of claims 7 to 10.

**Revendications**

1. Procédé de détermination et de sélection d'un plan d'irradiation (57, 59, 61 ; 57', 59', 61') pour la radiothérapie d'un patient, présentant les étapes suivantes :

   lors d'une première phase préalablement à une irradiation planifiée :

   - acquisition de plusieurs jeux de données de planification (43, 45, 47 ; 43', 45', 47'), dans lesquels un volume cible à irradier (51, 51') est représenté dans une position différente dans un objet cible, et
   - génération d'un plan d'irradiation (57, 59, 61) pour chacun de ces jeux de données de planification (41, 43, 45 ; 41', 43', 45'),

   lors d'une deuxième phase consécutive à la première phase préalablement à une irradiation planifiée :

   - enregistrement d'un jeu de données de vérification (41, 41'),
   - comparaison du jeu de données de vérification (41, 41') avec la pluralité de jeux de données de planification (43, 45, 47 ; 43', 45', 47') en ce qui concerne une similitude,
   - sélection d'un jeu de données de planification (43, 43') parmi la pluralité de jeux de données de planification (43, 45, 47 ; 43', 45', 47'), lequel présente la plus grande similitude avec le jeu de données de vérification (41, 41'),
   - sélection du plan d'irradiation (57, 57') qui est associé au jeu de données de planification (43, 43') sélectionné, et chargement de ce plan d'irradiation avec le dispositif de sélection,

   **caractérisé en ce qu'**une zone (73, 75) dans l'un des jeux de données de planification (43, 45, 47 ; 43', 45', 47') et/ou dans le jeu de données de vérification (41, 41') est sélectionnée, et dans lequel le jeu de données de vérification (41, 41') est comparé avec la pluralité de jeux de données de planification (43, 45, 47 ; 43', 45', 47') uniquement dans cette zone (73, 75) en ce qui concerne la similitude,
   **en ce que** la zone (73, 75) comprend le volume cible (51, 51') et au moins une zone (53, 55) adjacente au volume cible (51, 51'), et
   **en ce que** la zone (73, 75) ne comprend pas au moins une zone partielle (49, 56) dans l'un des jeux de données de planification (43, 45, 47 ; 43', 45', 47') et/ou dans le jeu de données de vérification (41, 41'), laquelle zone partielle (49, 56) n'a aucun point de contact avec le volume cible (51, 51').

2. Procédé selon la revendication 1, dans lequel la zone comprend un canal d'entrée (75) d'un rayon de traitement.

3. Procédé selon l'une des revendications 1 à 2, dans lequel la zone (73, 75) comprend en partie uniquement au moins une structure (53, 55) adjacente au volume cible (51, 51').

4. Procédé selon l'une des revendications 1 à 3, dans lequel la pluralité de jeux de données de planification (43', 45', 47') et/ou le jeu de données de vérification (41') comprennent une dimension temporelle.

5. Procédé selon l'une des revendications 1 à 4, dans lequel lors de la comparaison de la pluralité de jeux de données de planification (43, 45, 47 ; 43', 45', 47') avec le jeu de données de vérification (41, 41') une carte de sensibilité (81) est utilisée, qui caractérise une variabilité entre la pluralité de jeux de données de planification (43, 45, 47 ; 43', 45', 47') en ce qui concerne différentes régions (83, 85, 87).

6. Procédé selon l'une des revendications 1 à 5, dans lequel la génération des plans d'irradiation se produit lorsque les jeux de données de planification (43, 45, 47 ; 43', 45', 47') ont les mêmes instructions, notamment en ce qui concerne les marges de sécurité (63) à utiliser.

7. Procédé de détermination et de sélection d'un plan d'irradiation (57, 59, 61 ; 57', 59', 61') pour la radiothérapie d'un patient, présentant :

un dispositif de saisie (33), avec lequel un jeu de données de vérification (41, 41') et une pluralité de jeux de données de planification (43, 45, 47 ; 43', 45', 47') sont chargeables,

un dispositif de comparaison (35), avec lequel préalablement à une irradiation planifiée une comparaison du jeu de données de vérification (41, 41') avec la pluralité de jeux de données de planification (43, 45, 47 ; 43', 45', 47') peut être effectuée en ce qui concerne une similitude,

un dispositif d'évaluation (37), avec lequel préalablement à l'irradiation planifiée un jeu de données de planification (43, 43') peut être déterminé à partir de la pluralité de jeux de données de planification (43, 45, 47 ; 43', 45', 47'), lequel présente la plus grande similitude avec le jeu de données de vérification (41, 41'), et

un dispositif de sélection (39), avec lequel préalablement à l'irradiation prévue un plan d'irradiation (57, 57'), qui est associé au jeu de données de planification (43, 43') déterminé par le biais du dispositif d'évaluation (37), est chargeable,

caractérisé en ce qu'en outre le dispositif de comparaison (35) est configuré de telle sorte qu'une région (73, 75) peut être déterminée dans l'un des jeux de données de planification (43, 45, 47 ; 43', 45', 47') et/ou dans le jeu de données de vérification (41, 41'), dans lequel la comparaison du jeu de données de vérification (41, 41') avec la pluralité de jeux de données de planification (43, 45, 47 ; 43', 45', 47') s'effectue en ce qui concerne la similitude uniquement dans la zone (73, 75),

en ce que la zone (73, 75) comprend le volume cible (51, 51') et au moins une zone (53, 55) adjacente au volume cible (51, 51'), et

en ce que la zone (73, 75) ne comprend pas au moins une zone partielle (49, 56) dans l'un des jeux de données de planification (43, 45, 47 ; 43', 45', 47') et/ou dans le jeu de données de vérification (41, 41'), laquelle zone partielle (49, 56) n'a aucun point de contact avec le volume cible (51, 51').

8. Dispositif selon la revendication 7, dans lequel la zone comprend un canal d'entrée (75) d'un rayon de traitement.

9. Dispositif selon la revendication 7 ou 8, dans lequel le dispositif de comparaison (35) est configuré pour utiliser lors de la comparaison de la pluralité de jeux de données de planification (43, 45, 47 ; 43', 45', 47') avec le jeu de données de vérification (41, 41') une carte de sensibilité (83), laquelle caractérise une variabilité entre la pluralité de jeux de données de planification (43, 45, 47 ; 43', 45', 47') en ce qui concerne différentes régions (83, 85, 87).

10. Dispositif selon l'une des revendications 7 à 9, dans lequel en outre un dispositif de planification d'irradiation (31) est prévu, avec lequel respectivement un plan d'irradiation (57, 59, 61 ; 57', 59', 61') peut être généré pour les jeux de données de planification (43, 45, 47 ; 43', 45', 47').

11. Installation d'irradiation (10) avec un dispositif selon l'une des revendications 7 à 10.

# FIG 1

EP 2 158 939 B1

# FIG 2

33

35

37

39

31

FIG 3

FIG 4

## FIG 5

## FIG 6

# FIG 7

**IN DER BESCHREIBUNG AUFGEFÜHRTE DOKUMENTE**

**In der Beschreibung aufgeführte Patentdokumente**

- US 20050201516 A1
- US 20070076846 A

**In der Beschreibung aufgeführte Nicht-Patentliteratur**

- **VON NIKOGHOSYAN A.** *Evaluation of the therapeutical potential of heavy ion therapy for patients with locally advanced prostate cancer,* Oktober 2004